# EUROPEAN PATENT SPECIFICATION

(11) **EP 4 440 458 B1**
(45) Date of publication and mention of the grant of the patent: **29.10.2025**
(21) Application number: 22902269.4
(22) Date of filing: 02.12.2022
(51) Int. Cl.: A61B 18/14, A61B 18/00, A61B 90/00, A61M 3/00

(54) **DEVICES FOR TREATING LUNG TUMORS**
VORRICHTUNGEN ZUR BEHANDLUNG VON LUNGENTUMOREN
DISPOSITIFS POUR LE TRAITEMENT DU CANCER DU POUMON

(30) Priority: 03.12.2021 US 202163285982 P; 19.12.2021 US 202163291428 P; 12.05.2022 US 202263341359 P; 11.06.2022 US 202263351384 P; 12.06.2022 US 202263351430 P; 06.07.2022 US 202263358806 P; 10.08.2022 EP 22189768; 19.10.2022 US 202263417600 P
(43) Date of publication of application: 09.10.2024
(73) Proprietor: Tau Medical Inc., Centreville, Virginia 20120 (US); Institute For Research & Industry Cooperation Pusan National University, Busan, Gyeongsangnam-do 46241 (KR); Tau Medical Inc., Busan, Gyeongsangnam-do 46285 (KR)
(72) Inventor: KIM, June-Hong, Busan, Gyeongsangnam-do 48516 (KR)
(74) Representative: FRKelly
(86) International application number: PCT/US2022/051727
(87) International publication number: WO 2023/102235

(56) References cited:
- WO-A1-2019/051251
- WO-A1-2021/226130
- US-A- 5 248 312
- US-A1- 2005 288 684
- US-A1- 2007 167 944
- US-A1- 2010 152 724
- US-A1- 2012 289 956
- US-A1- 2016 249 972
- US-A1- 2021 068 895
- BO GUYUE, REN LONG, XU XUN, DU YI, DOU SHIXUE: "Recent progress on liquid metals and their applications", ADVANCES IN PHYSICS: X, vol. 3, no. 1, 1 January 2018 (2018-01-01), pages 1446359, XP093072297, DOI: 10.1080/23746149.2018.1446359

## Description

### Technical Field

The present disclosure is directed generally to devices and methods for ablating lung tumors and more particularly the ablation treatment of lung tumors by using liquid metal as an electrode.

### Background

To date, thermal ablation is becoming an increasingly attractive choice for the treatment of unresectable tumors in the lung. Radiofrequency (RF) energy has seen the most utility for pulmonary ablation. However, there are significant technical deficiencies in the delivery of RF energy to the lung. The use of multi prong electrodes, which increase total electrode surface area, and ionic fluid infusion have been shown to decrease impedance to RF current flow in the lung. Although effective, these techniques are not without drawbacks. Fluid infusion is unpredictable and has been associated with an increased risk of complications. Multitined electrodes increase invasiveness, can be difficult to use in solid tumors situated in normal lung tissue, and have been associated with irregular zones of ablation and increased rates of occurrence of pneumothorax.

In addition, various radiofrequency ablation (RFA) has been utilized for treating peripheral lung tumors. However, there remains a need for improvement due to insufficient ablation coverage and the difficulty of endoscopically navigating the ablation electrodes to targeted tumors in the peripheral pulmonary lesions (PPL). It is desired for the ablation electrodes to be flexible and relatively soft and fit in the PPL that is small in diameter, preferably less than 2 mm, to ablate tumors that are closer to the peripheral of the lung.

Although hyperthermic treatment using radio frequency (RF) ablation is a treatment for lung tumors, the requirement of exact delivery of the rigid metal electrode into the center of the target site in the peripheral lung legion often may result in the risk of damage to the surrounding regions or suboptimal ablation. WO2019051251 A1 is directed generally to devices and methods for ablating malignant lung tumors and more particularly to ablating lung tumors with an approach through the patient's airway.

### Summary

The invention is defined in claim 1. Any methods disclosed hereinafter do not form part of the scope of the invention, and are mentioned for illustrative purposes only. To solve the problems of the requirement of exact delivery of the rigid metal electrode into the center of a target site, an RF ablation catheter device uses a medical grade liquid metal device instead of using a solid electrode. The liquid metal device acts as an electrode through which RF ablation energy can be applied against the lung tumors. By injecting the liquid metal device into a target site, this will be conforming with the anatomical structures of the target site. Because of this conforming shape of the liquid metal device, there is less risk of damage to the surrounding regions. The liquid metal device is easily removed by aspiration suction without damaging the surrounding regions. Accordingly, the liquid metal device acts an independent and flexible electrode within the target site creating larger ablation area.

The RF ablation catheter device configured to ablate tumors adjacent the target site using a liquid metal device comprises a flexible shaft configured to advance endobronchially into the target site. The catheter device further comprises an inflatable balloon mounted on the shaft wherein a portion of the flexible shaft that is distal to the inflatable balloon is defined as a distal segment. The catheter device further comprises a RF conductor attached to the distal segment and configured to allow RF current to pass through the liquid metal device in such a way the liquid metal device delivers radio frequency (RF) energy to ablate the tumors.

In one aspect, a radiofrequency (RF) ablation catheter configured to heat a passageway filled with a liquid metal, the ablation catheter comprises a flexible shaft extending between a distal end and a proximal end. The flexible shaft has a fluid channel configured to instill the liquid metal into the passageway. The ablation catheter further comprises an inflatable balloon mounted on the flexible shaft. The ablation catheter further comprises an RF connector. A portion of the flexible shaft that is distal to the inflatable balloon is defined as a distal segment of the flexible shaft. The RF connector is located at the distal segment and is configured for connecting RF energy to the liquid metal thereby heating the passageway.

In the RF ablation catheter the distal segment forms a conduit inside the distal segment in such a way the conduit conveys the liquid metal from the fluid channel. In one embodiment, the RF connector is located within the conduit in a recess manner. In one embodiment, the RF connector is arranged at a proximal end of the distal segment directly neighboring the inflating balloon. In one embodiment, the RF connector is an electrically conductive wire connected to a RF generator. The ablation catheter further comprises a temperature sensor mounted on the distal segment of the shaft and configured to read the temperature of the liquid metal. The temperature sensor is arranged within the conduit in a recessed manner. The conduit is made of non-conductive material. Herein the passageway is a bronchial airway of the lung.

In another aspect, an ablation catheter assembly configured to heat a passageway, the assembly comprises a liquid metal contained in a syringe. The ablation assembly further comprises an RF ablation catheter comprising a flexible shaft having a fluid channel to instill the liquid metal into the passageway; an inflatable balloon mounted on the flexible shaft and a RF connector. Herein a portion of the flexible shaft that is distal to the inflatable balloon is defined as a distal segment of the flexible shaft. Also, herein the RF connector is located at the distal segment and is configured for connecting RF energy to the liquid metal thereby heating the passageway.

The ablation catheter assembly further comprises a flexible bronchoscope comprising a working channel configured for the ablation catheter to travel through the working channel. The ablation catheter assembly further comprises an RF generator electrically coupled to the RF connector. Herein, the passageway is a bronchial airway of the lung. In the ablation catheter assembly, the liquid metal comprises a gallium, and the liquid metal is E-Galn.

In the ablation catheter assembly, the RF ablation catheter does not use a solid electrode for ablation. In the RF ablation catheter, the distal segment forms a conduit inside the distal segment in such a way the conduit conveys the liquid metal from the fluid channel. In some embodiments, the RF connector is located within the conduit in a recessed manner. In some embodiments, the conduit is made of non-conductive material.

A method of treating a tumor in a patient, comprising (a) inserting an ablation catheter into a passageway; (b) advancing the ablation catheter device to a passageway; (c) inflating the balloon to obstruct a passageway (d) instilling a liquid metal into a passageway; and (e) applying an RF current to a liquid metal in a passageway.

In the above method the passageway is a bronchial airway of the lung. The method further comprises instilling the liquid metal into at least two branches of the bronchial airway. In the above method, alveoli of the bronchial airway are not filled with the liquid metal.

The above method further comprises (a) having a flexible bronchoscope that comprises an instrument channel; (b) inserting the ablation catheter through the instrument channel of the bronchoscope; (c) advancing the bronchoscope to the passageway; (d) advancing the ablation catheter out of the instrument channel of the bronchoscope and into the passageway, wherein the passageway is a bronchial airway of the lung.

The above method further comprises aspirating the liquid metal out of the passageway by suction through the bronchoscope. The above method further comprises visualizing the ablation catheter by x-ray fluoroscopy while advancing the ablation catheter to the passageway. The above method further comprises visualizing the liquid metal by x-ray fluoroscopy while instilling the liquid metal into the passageway.

In the above method, the volume of the liquid metal instilled is less than 1.0 ml. Also, the liquid metal comprises gallium. In some embodiments, the liquid metal is E-Galn. In the above method, the liquid metal is instilled only into bronchial airways having a diameter of less than 5 mm. In some embodiments, the liquid metal is instilled only into bronchial airways having a length of less than 10 cm.

### BRIEF DESCRIPTION OF THE DRAWINGS

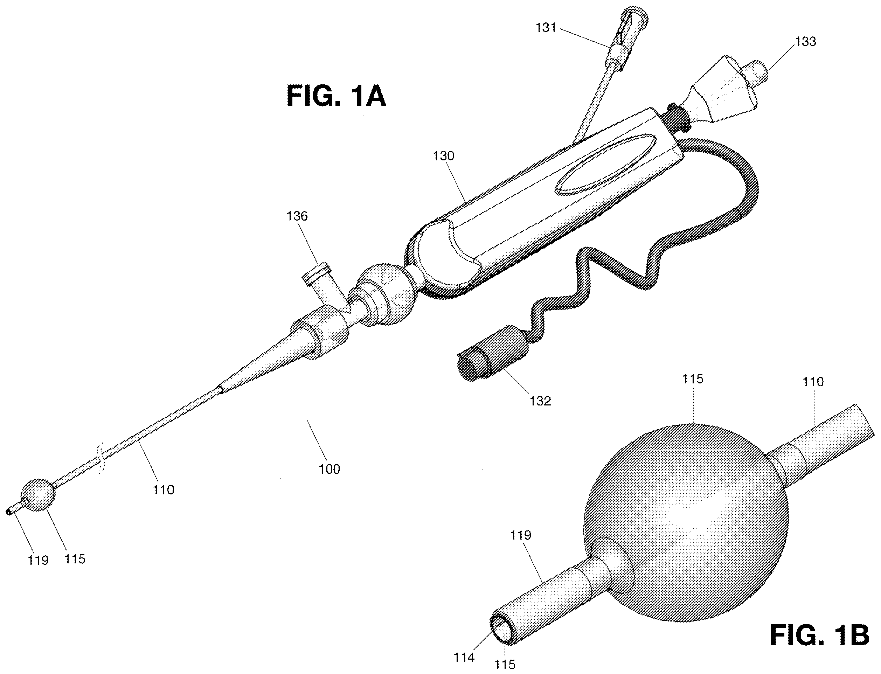

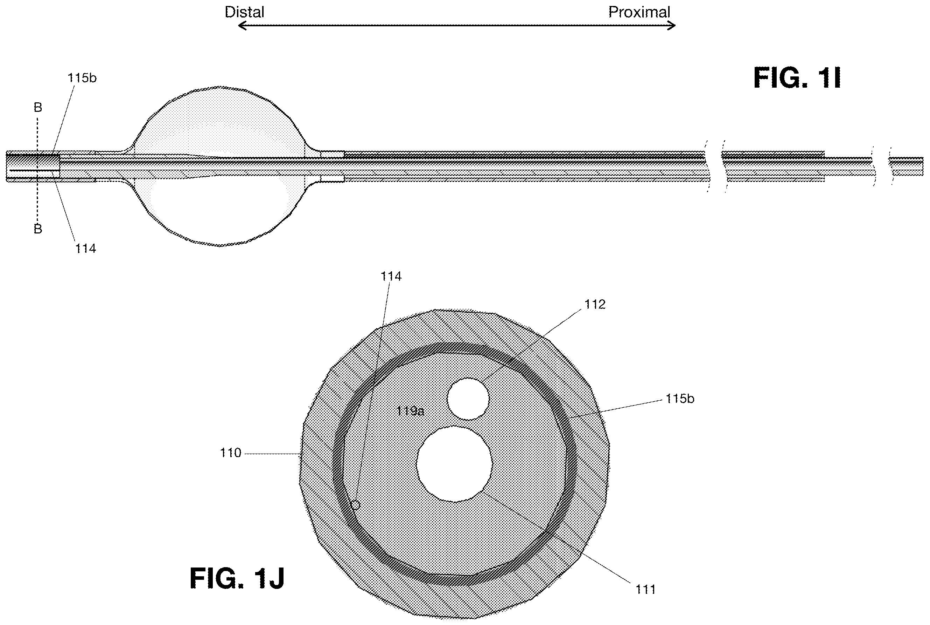

FIGS. 1A-1B show a perspective view of the ablation catheter.

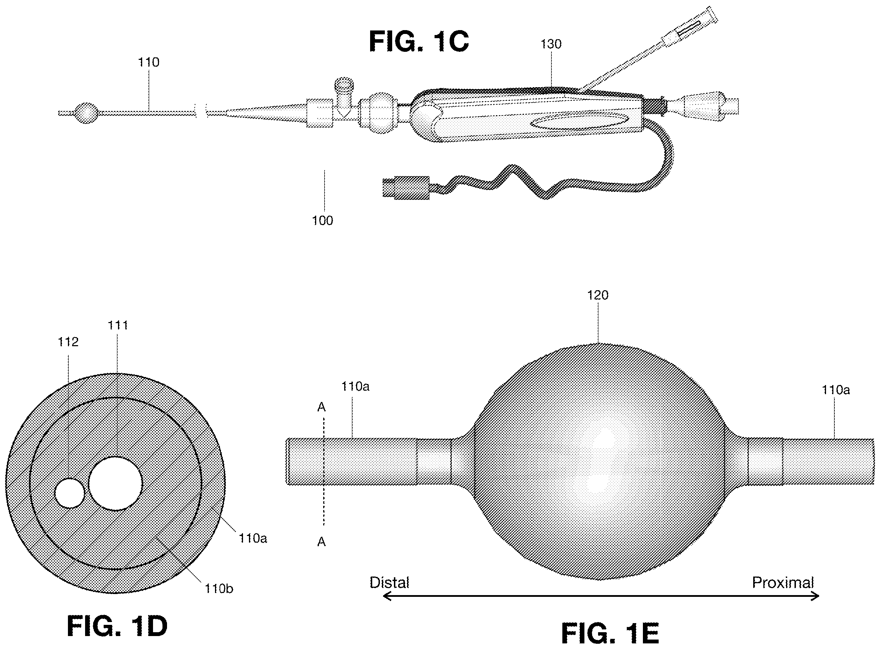

FIG. 1C shows a side view of the ablation catheter.
FIG. 1D shows a cross-sectional view of A-A of FIG. 1E.
FIG. 1E shows a side view of the occlusion balloon of the ablation catheter.

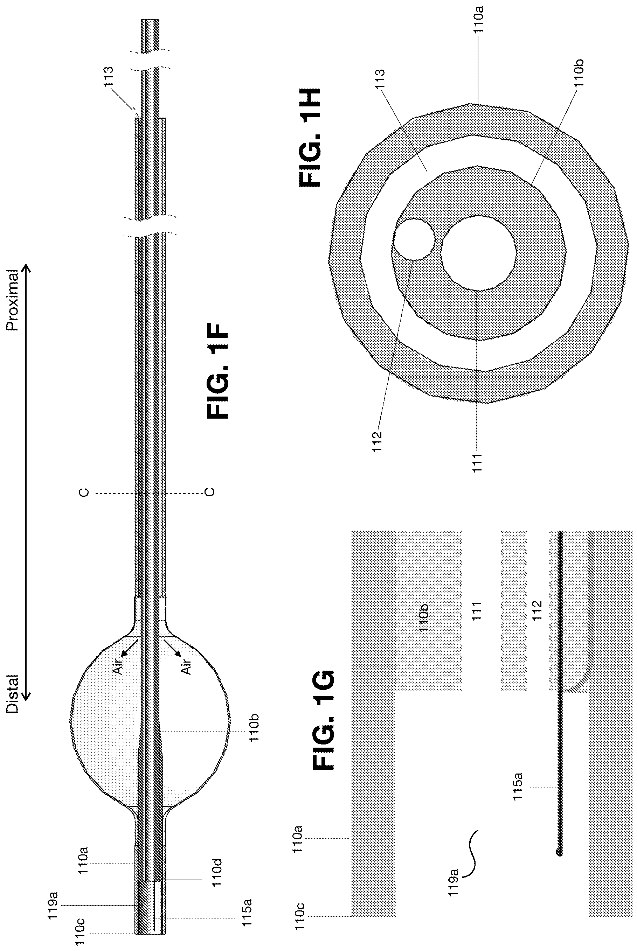

FIG. 1F shows a cross-sectional view of the flexible shaft.
FIG. 1G shows an expanded cross-section view of the distal portion of FIG. 1F.
FIG. 1H shows a cross-sectional view cut at C-C of FIG.1F.
FIG. 1l shows a cross-section view of the flexible shaft.
FIG. 1J shows a cross-section view cut at B-B of FIG. 1l.
FIGS. 2A-2D show cutaway views of different embodiments of the catheter.
FIG. 2E showing a schematic view of the parts of an example catheter.
FIGS. 2F-2l showing different embodiments of a catheter having a guidewire.
FIG. 3 shows the ablation catheter assembly.
FIG. 4A shows the ablation catheter assembly placed in the lung.
FIG. 4B shows the preferred target site for ablation of the lung.
FIG. 4C shows the preferred target site and sensitivity zone.
FIG. 4D shows the target airway filled with the liquid metal.
FIG. 4E shows the target airway when the liquid metal is removed.
FIG. 5A shows the target ablation site in the peripheral legion.
FIG. 5B shows the preferred ablation zone.
FIG. 5C(a) shows the liquid metal is filled in the target airway.

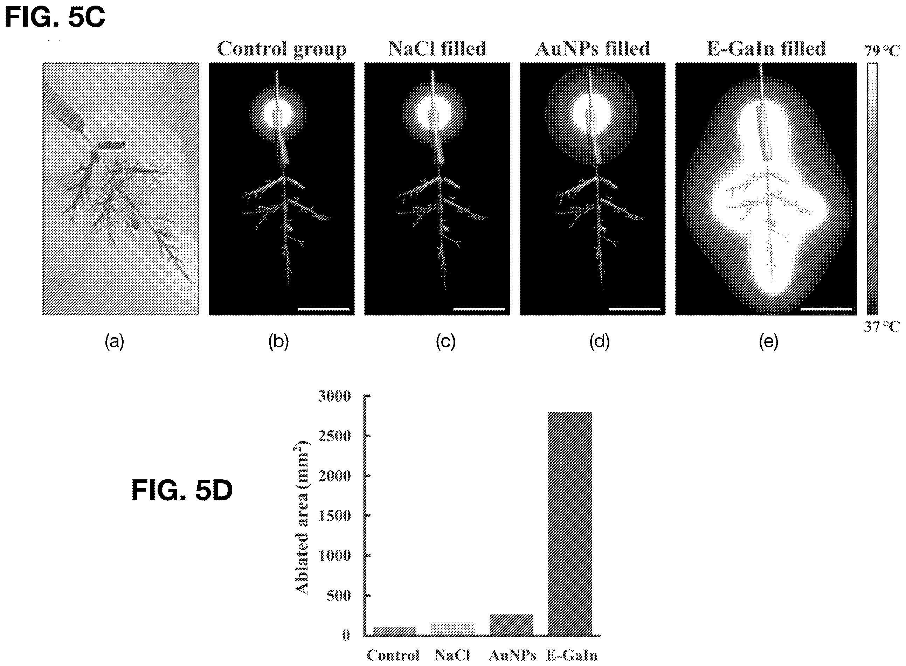

FIG. 5C(b) shows the ablation size when nothing is filled in the target airway.
FIG. 5C(c) shows the ablation size when NaCl is filled in the target airway.
FIG. 5C(d) shows the ablation size when AuNP is filled in the target airway.
FIG. 5C(e) shows the ablation size when EGaln is filled in the target airway.
FIG. 5D shows a comparison chart showing the ablation area size.

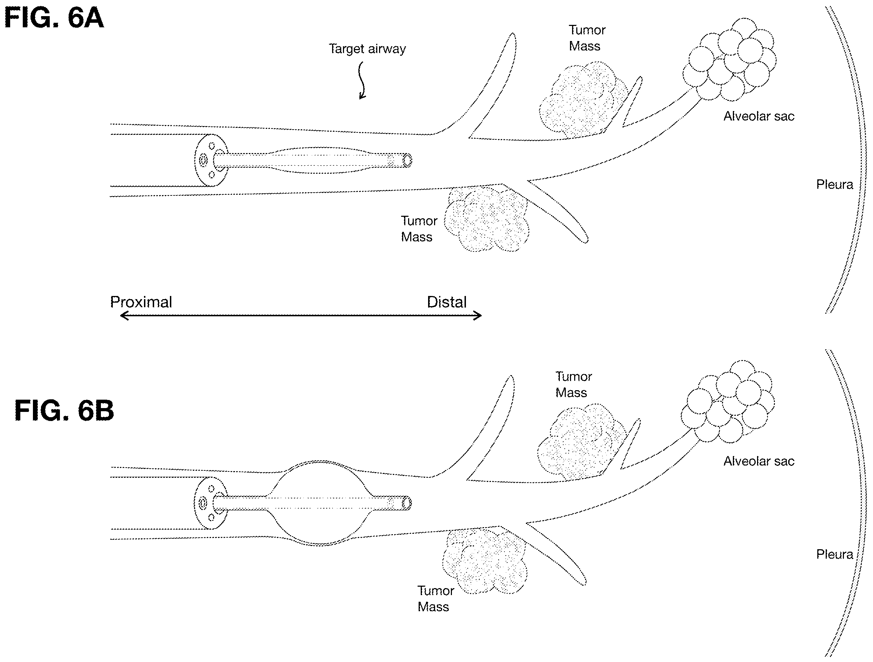

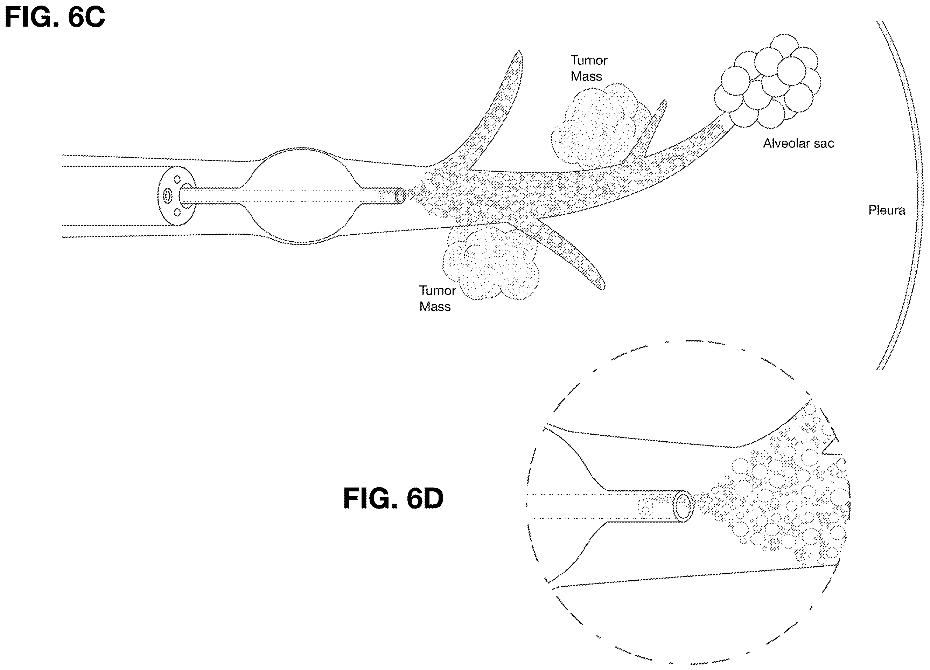

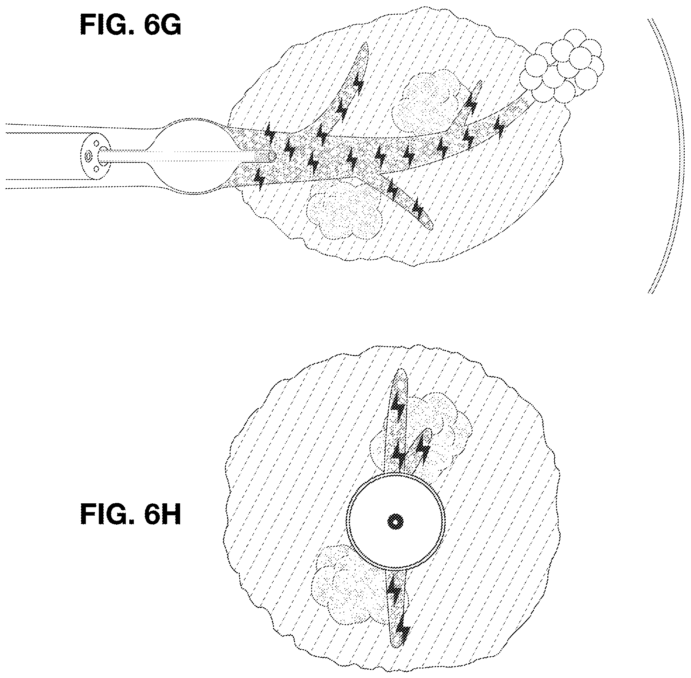

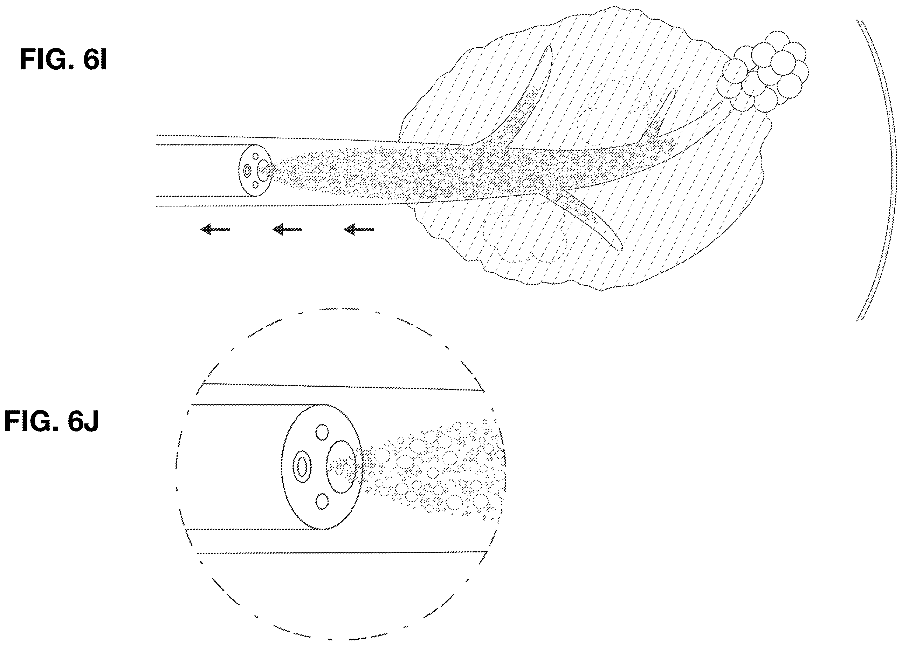

FIGS. 6A-6B show the steps of the ablation catheter positioned at the target site.
FIGS. 6C-6D show the steps of instilling the liquid metal into the target airway.

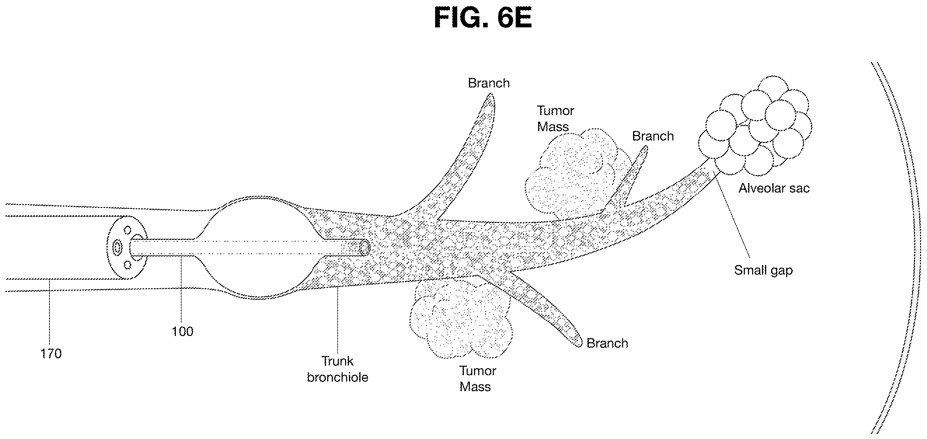

FIG. 6E shows the step of completing the instilling of the liquid metal.

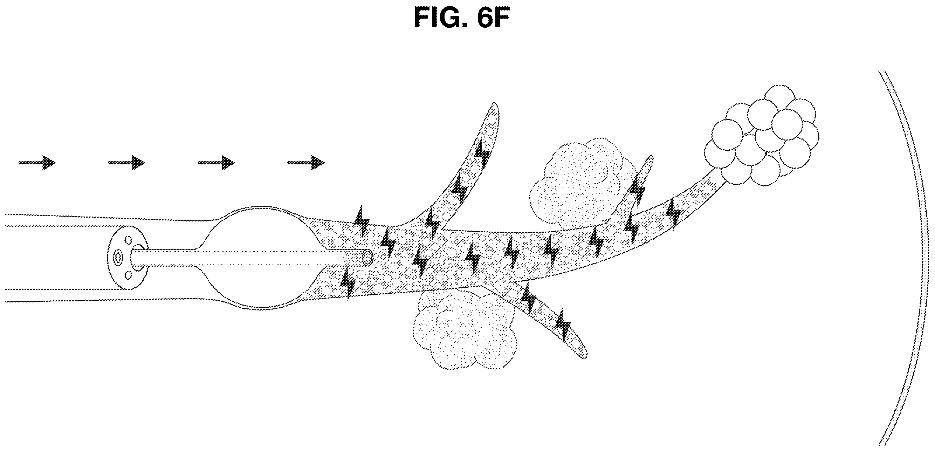

FIG. 6F shows the step of applying the RF current to the liquid metal.
FIGS. 6G-6H show the situation where the target zone is being ablated.
FIGS. 6l-6J show the step of suctioning of the instilled liquid metal.

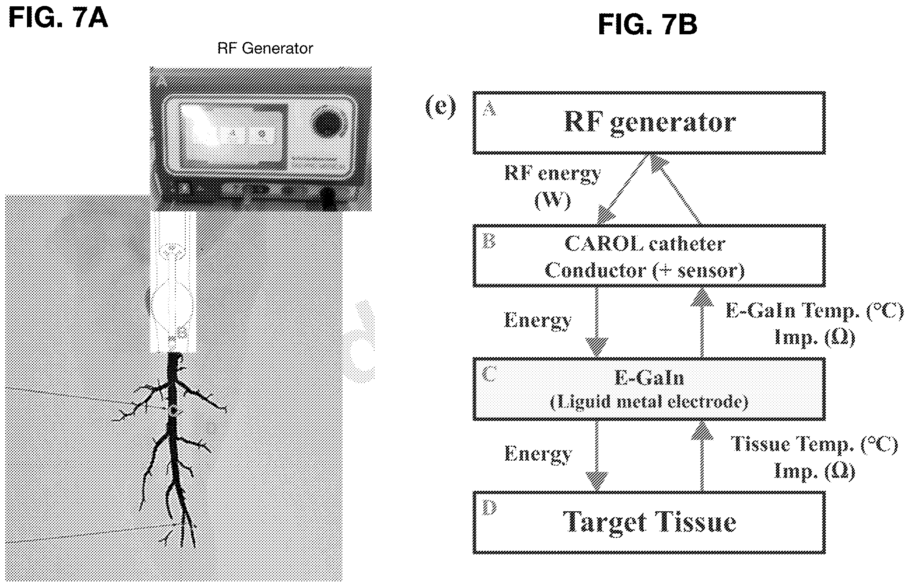

FIGS. 7A-7B show the operating parameter and flow chart.

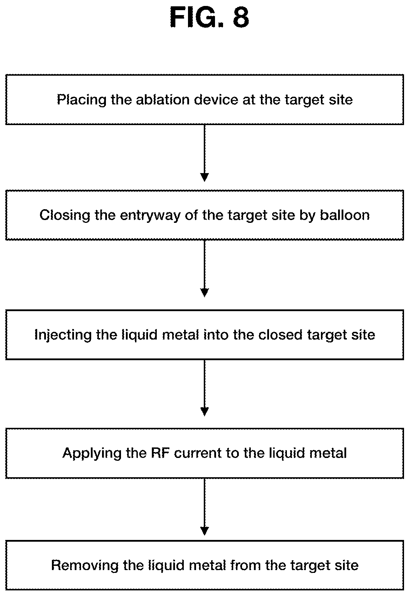

FIG. 8 shows an exemplified steps of the procedure.

### DESCRIPTION OF EXAMPLE EMBODIMENTS

To assist in understanding the invention, reference is made to the accompanying drawings to show by way of illustration specific embodiments in which the invention may be practiced. The drawings herein are not necessarily made to scale or actual proportions. For example, the lengths and widths of the components may be adjusted to accommodate the page size.

This disclosure is related to methods, devices, assemblies, and system for transbronchial ablation of a lung tumor. Aspects of the disclosure are included as follows. A radiofrequency (RF) ablation catheter which is configured to heat a passageway filled with a liquid metal, the ablation catheter comprises a flexible shaft having a proximal end and a distal end, the flexible shaft having a fluid channel configured to instill the liquid metal into the passageway. The ablation catheter further comprises an inflatable balloon mounted on the flexible shaft wherein a portion of the flexible shaft that is distal to the balloon is defined as a distal segment of the flexible shaft, the balloon configured to obstruct the passageway. The ablation catheter comprises an RF conductor located on the distal segment configured for connecting RF energy to the liquid metal thereby heating the passageway.

### Target Site

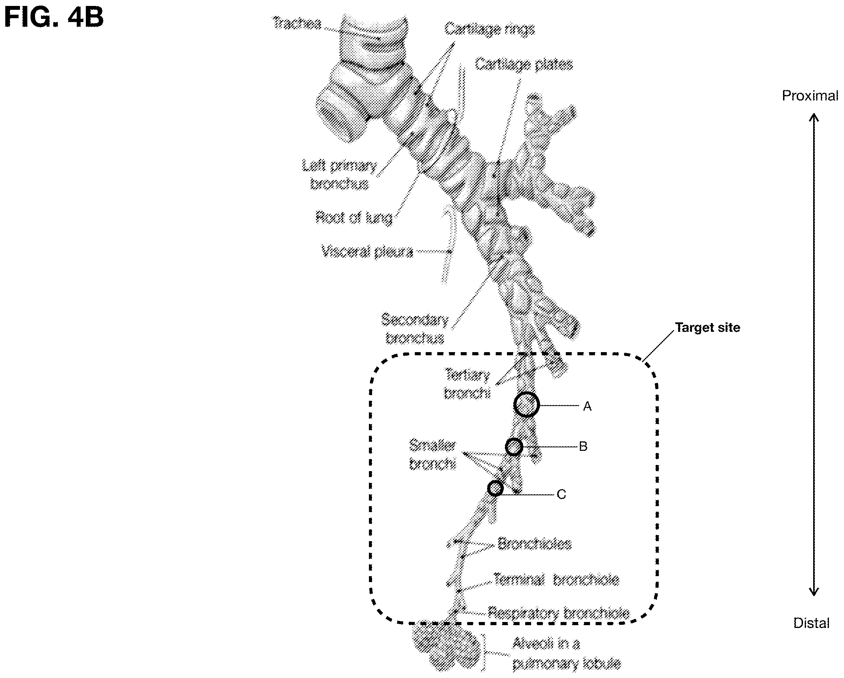

Throughout this disclosure, the target site or region may be referred to the bronchial trees located between the segmental bronchus (3^{rd}-4^{th} generations), subsegmental bronchus (5^{th}-11^{th} generations), bronchioles (12^{th}-15^{th} generations), and terminal bronchioles (16^{th} generation). The preferred target site may further include the respiratory bronchioles (17^{th}-9^{th} generations). FIGS. 4B-4C, for example, show the examples of the target site comprising tertiary bronchi, smaller bronchi, bronchioles, terminal bronchiole, and respiratory bronchiole. In some embodiments applicable to body organ other than the lung, the target site could be performed in the hepatobiliary duct or the pancreatic duct.

### Target Airway

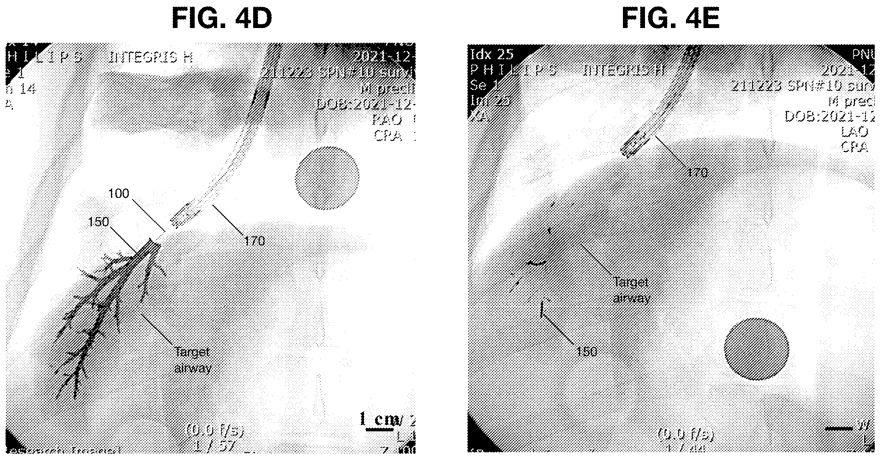

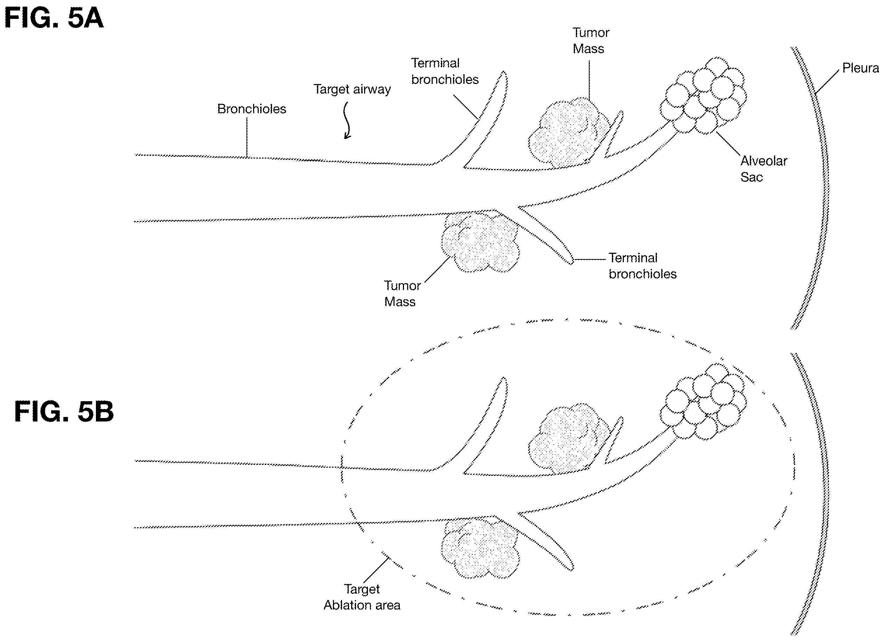

Also, throughout this disclosure, the target airway or passageway is referred to bronchial airways surrounding or adjacent target tumor masses within the target site. Note that the target airway includes main bronchial airway and its subbranches as shown in FIGS. 4D and 5A-5B. The main bronchial airway may have a tapered shape having a proximal end. In one embodiment, the target airway may have a diameter of less than 0.5 cm at its proximal end. In some embodiments, the target airway may have a length of less than 6 cm. The preferred target airway may be within the peripheral airways because the target airway's diameter and length has a close relationship with the ablation size.

Once the target airway is determined, the operator may determine one of the occlusion balloon positions (i.e., A, B, or C) located at its proximal portion of the target airway as exemplified in FIG. 4B within the target site for closing the selected target site. By instilling the liquid metal into the closed target site, the instilled liquid metal conforms with anatomical structures of the main airway and its subbranches, and acts an electrode through which RF energy can be applied against tumors.

### Flexible Shaft

In one embodiment, the ablation catheter 100 comprises the flexible shaft 110. FIG. 1A shows the flexible shaft 110 has the occlusion balloon 120 mounted on the distal portion and a handle portion 130 attached to the proximal portion. FIG. 1B shows an expanded view of the distal portion of the shaft 110 showing the balloon 120. In one embodiment, the flexible shaft has a length of 50-250 cm.

In one embodiment, the flexible shaft 110 further comprises an outer shaft 110a and an inner shaft 110b which have the distal ends 110c and 110d respectively. The outer shaft 110a has a lumen for insertion of the inner shaft 110b as shown in FIG. 1F. In some embodiments, the outer shaft 110a has an outer diameter of less than 1.65 mm.

In one embodiment, the inner shaft 110b comprises a guidewire lumen 111 having an outer diameter of less than 0.5 mm for insertion of a guidewire. The inner shaft 110b further comprises a fluid channel 112 having an outer diameter of less than 0.3 mm as shown in the cross-sectional view (C-C) of FIG. 1G. The guidewire lumen 111 is configured for insertion of the guidewire. In some embodiments, the fluid channel 112 is configured for passage (i.e., instilling or aspiration) of the liquid metal.

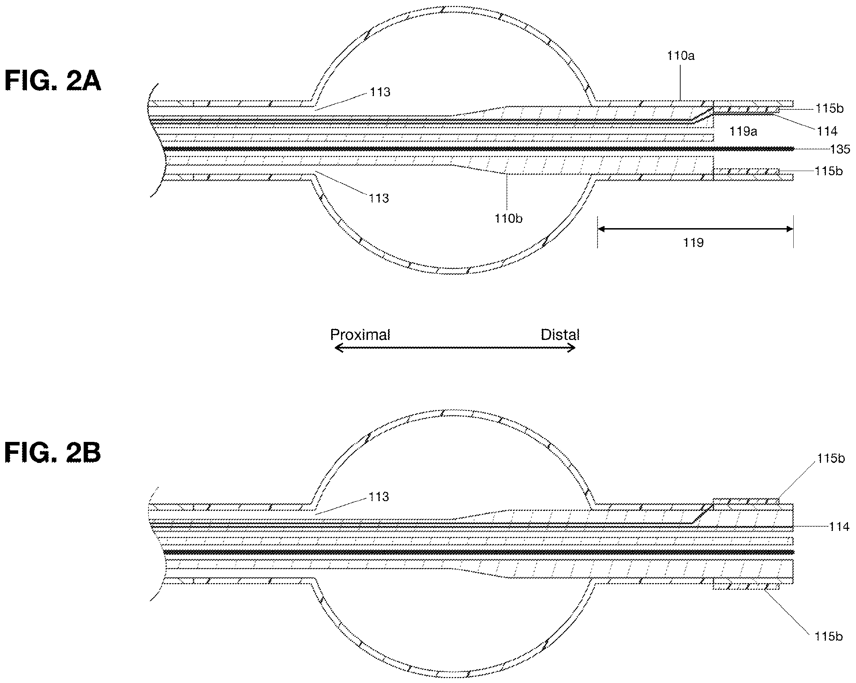

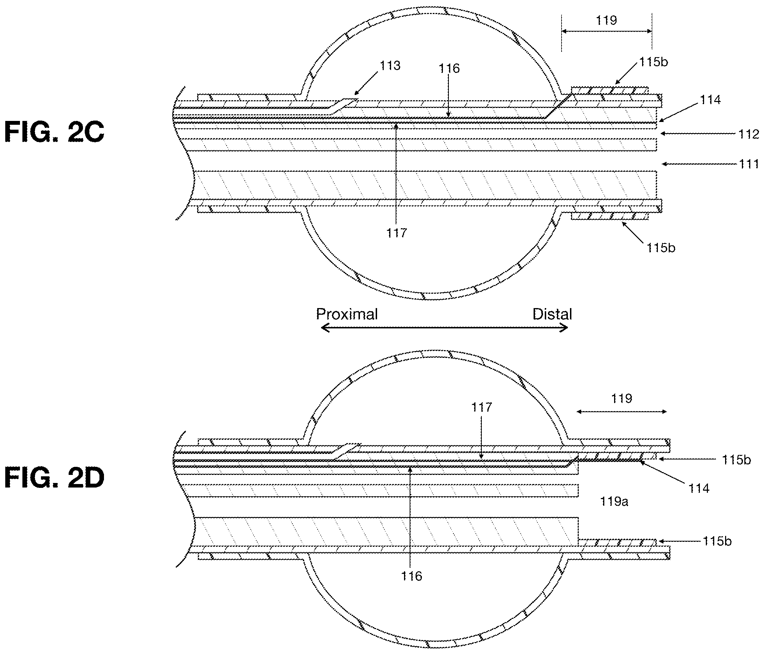

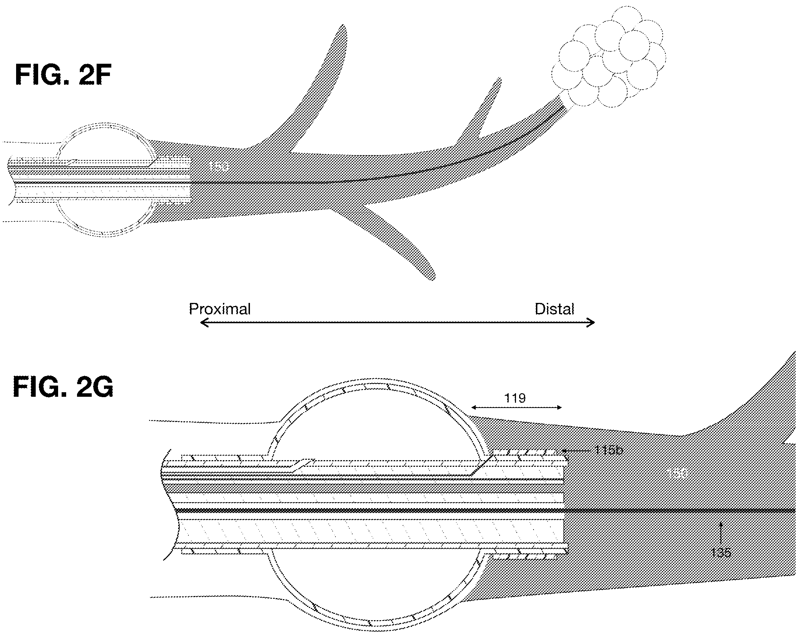

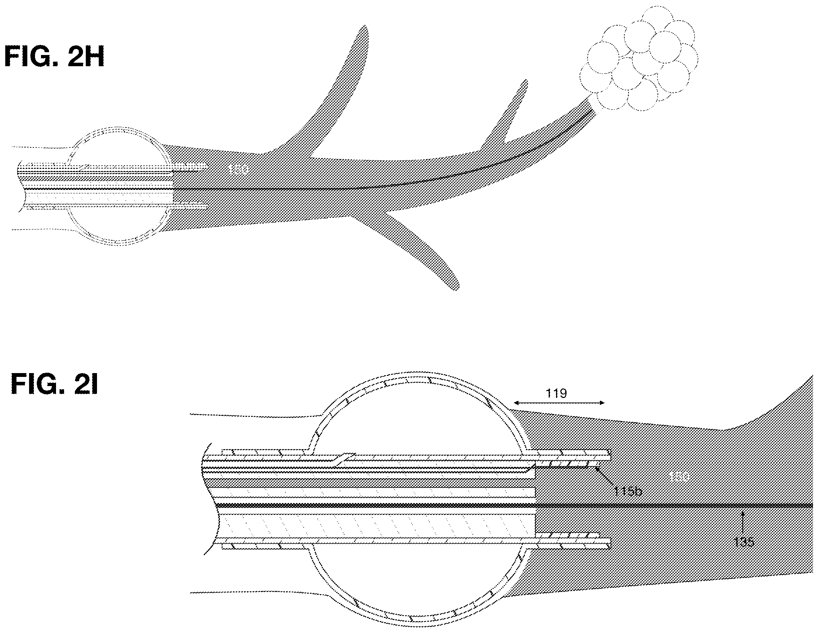

In one embodiment, as shown in FIGS. 1F and 1G, the distal end 110d of the inner shaft 110b may be arranged to have a distance from the distal end 110c of the outer shaft 110a. The inner space created from the distance between the outer and inner shafts is defined as a conduit (cavity) 119a. Preferably, as shown in FIGS. 2A and 2D, the conduit 119a creates a protected or insulated channel from the target airway tissue in such a way the liquid metal within the conduit 119 or cavity can avoid discontinuity of the liquid metal and direct touch with nearby airway tissues when RF energy is applied thereto. In some embodiments, the surface of the conduit 119a is made of electrically insulated material.

In one embodiment, the distal portion of the inner shaft 110b may be getting thicker so as to securely attach to the inside lumen of the outer shaft 110a while the proximal portion is getting thinner to create an inflation lumen 113. The inflation lumen 113 is defined the space between the outer shaft 110a and inner shaft 110b as shown in FIGS. 1F and 1G for the occlusion balloon 120 mounted on the outer shaft 110a for inflation or deflation of the balloon during the procedure. In some embodiments, as shown in FIGS. 2C-2D, an independent inflation lumen 113 may be constructed along the flexible shaft. The length and size of the conduit 119a can vary depending on the anatomical structure of the target airway. In some embodiments, the length of the conduit 119a may be less than 0.5 cm.

In some embodiments, the flexible shaft 110 can comprise an inner shaft 110b and an outer shaft 110a, wherein the outer shaft is arranged surrounding the inner shaft 110b to cover at least a partial length of the inner shaft. The flexible shaft 110 extends between the distal end to the proximal end along a shaft axis wherein the length of the shaft is defined along the shaft length axis.

### Occlusion Balloon

In one embodiment, the ablation catheter device 100 further comprises the occlusion balloon 120 mounted on the outer shaft 110a as shown in FIGS. 1F and 1H. The balloon 120 is located within 4-6 mm from the distal end 110c of the outer shaft 110a. In some embodiments, the ablation catheter 100 may have multiple occlusion balloons (now shown) depending on the anatomical structure of the target airway. In some embodiments, the occlusion balloon can work with other balloons equipped on a conventional bronchoscope.

In one embodiment, a portion of the outer shaft 110a that is distal to the occlusion balloon 120 can be defined as the distal segment 119 of the shaft as shown in FIG. 1B. In some embodiments, the distal segment 119 of the shaft has a length of less than 5.0 mm to avoid direct contact with the target airway tissue.

In some embodiments, the primary function of the occlusion balloon 120 is to close the entry way of the target airway which is to be filled with the liquid metal 150. In some embodiments, the entry way of the target airway, for example, could be about 4-5 mm in diameter. When the ablation catheter 100 is placed at the target airway, the balloon 120 is configured to close the entry way of the target airway before instilling the liquid metal. After the completion of the intended ablation, the balloon 120 is deflated and the ablation catheter 100 is removed from the bronchoscope 170 through the working channel.

In some embodiments, the flexible shaft 110 can further comprise an inflation lumen 113 defined by a space between the outer flexible shaft and the inner flexible shaft as shown in FIGS. 1F and 1H, in communication with the occlusion balloon 120 and adapted to supply or discharge a fluid to/from the inflatable/occlusion balloon 120. The inner shaft 110b can further comprise a guidewire lumen adapted for insertion of a guidewire. The guidewire can be formed of an electrical conductive material.

In some embodiments, the inner shaft 110b and the outer shaft 110a can have the same length distal to the inflatable balloon 120 to together form the distal end of the shaft and wherein the RF connector 115 is formed on the distal segment of the outer shaft 110a.

### RF Connector

In one embodiment, the ablation catheter further comprises a RF connector 115. The main function of the connector 115 is to connect RF energy to the liquid metal 150 thereby heating the target airway. Various forms of the RF connector 115 can be used. For example, in one embodiment, a conductive wire type RF connector 115b can be used as shown in FIG. 1G. Herein, the conductive wire RF connector 115a runs through the inner shaft 110b, and the distal portion of the conductive wire RF connector 115a is protruded from the distal end 110d of the inner shaft. The protruded portion of the conductive wire RF connector115a is arranged within the conduit 119a in such a way the conductive wire RF connector115a can connect RF energy to the liquid metal within the conduit 119a to avoid any discontinuity of the liquid metal and to avoid direct contact with the target airway tissues.

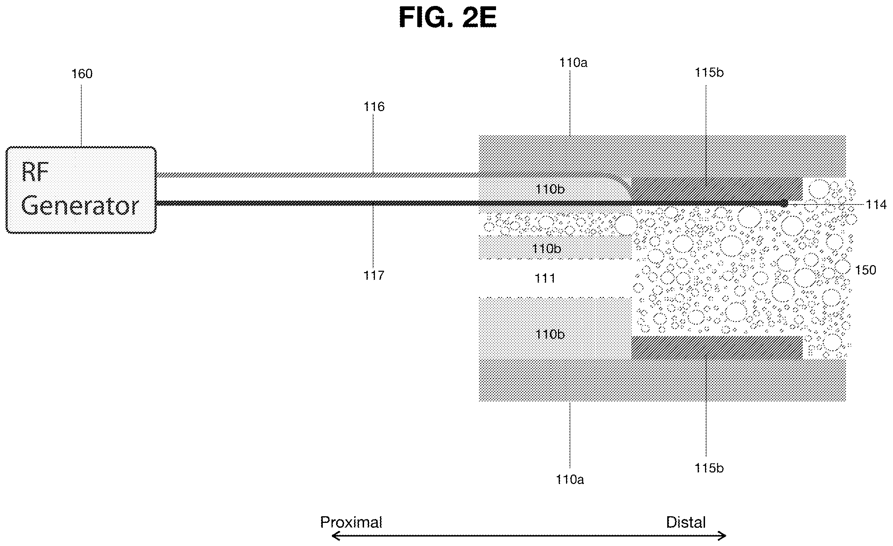

Alternatively, as shown in FIGS. 1l and 1J, a ring-shaped connector 115b can be used as shown in FIG.1J. The ring-shaped connector 115b that is made of conductive material is arranged into the conduit 119a in such a way the ring-shaped connector 115b connects RF energy to the liquid metal within the ring-shaped connector 115b without touching the target airway tissues. FIG. 1J shows a cross-section view cut at B-B of FIG.1H wherein the conduit 119a is illustrated within the ring-shaped connector 115b. In some embodiments, the ring-shaped connector 115b is connected to the conductive wire 116 which is connected to the generator via the inner shaft 110b as shown in FIG. 2E.

Alternatively, in some embodiments, the ring-shaped RF connector 115b can be attached on the surface of the distal segment 119 as shown in FIGS. 2B and 2C. In this case of avoiding the tissue contact, the preferred embodiment is that the ring-shaped RF connector 115b may be attached as close to the distal end of the balloon 120 as shown in FIG. 2C in such a way the ring-shaped RF connector 115b can avoid the direct contact with the tissue as shown in FIG. 2G when the RF current starts to run to the liquid metal 150. In some embodiments, the ring-shaped RF connector 115b is arranged at a proximal end of the distal segment 119 directly neighboring the occlusion balloon 120.

In some embodiments, the ring-shaped RF connector 115b can be arranged in a recessed manner in the conduit or cavity 119a to cover a partial surface area of said cavity. Alternatively, the RF connector can be arranged in the cavity to entirely cover the surface area of the cavity. As a further alternative, the ring-shaped RF connector 115b can be arranged in the cavity and protrudes via the at least one opening to the outside of the cavity 119a. The ring-shaped RF connector 115b which extends from the inside of the cavity to the outside of the cavity can cover the entire surface area of the cavity or just a partial surface area of said cavity.

In some embodiments, the ring-shaped RF connector 115b can be formed by at least one electrically conductive metal element on the distal segment 119 of the shaft 110. In some embodiments, the at least one electrical conductive element can be formed as a ring-segment to cover at least one/a proportion of the circumference of the surface area of the distal segment or can be alternatively formed as an entire ring to cover the whole circumference of a partial surface area of the distal segment 119.

In some embodiments, the at least one electrically conductive element can be formed pad shaped or formed by at least one conductive wire. Furthermore, according to the invention, the conductive element can be formed by an electrically conductive metal mesh. In some embodiments, the ring-shaped RF connector 115b can be arranged at a proximal end of the distal segment directly neighboring the inflatable balloon 120.

In some embodiments, the ring-shaped RF connector 115b can be adapted to electrically be connected to an RF generator and to pass a flow of an RF current from the RF generator to the liquid metal. The catheter can further comprise an RF generator which is electrically coupled to the ring-shaped RF connector 115b.

In some embodiments, the flexible shaft 110 can further comprise a guidewire lumen adapted to receive a guidewire. In some embodiments, the guidewire can be made of an electrically conductive material in such a way the guidewire may be configured to electrically connect the discontinuity of the liquid metal within the target airway.

Alternatively, the length of the inner shaft 110b distal to the inflatable balloon 120 can be formed shorter than the length of the outer shaft 110a distal to the balloon to form a cavity between the distal end of the outer shaft and the distal end of the inner shaft. The cavity has a distal opening and the ring-shaped RF connector 115b is arranged in said cavity.

In some embodiments, the inflatable balloon can be mounted on the outer shaft of the inflatable balloon and can be integrally formed with the outer shaft. The distal segment of the flexible shaft comprises a cover element to partially overlay a conductive surface of the ring-shaped RF connector 115b, wherein the cover element is made of a non-conductive material.

In some embodiments, the distal segment 119 can comprise a cover element which is attached to the distal segment of the shaft to form a cavity with at least one opening, wherein the ring-shaped connector 115b is received in said cavity and wherein the cover element is made of a non-conductive element.

In some embodiments, the cover element can be formed as a hollow cylinder to surround a partial length of the distal segment of the shaft and to form a cavity with at least an opening wherein the cavity extends from the distal end of the shaft.

### Temperature Sensor

In one embodiment, the ablation catheter 100 further comprises the temperature sensor 114 configured to read the temperature of the liquid metal. The temperature sensor 114 is connected to a thermocouple 117 which runs through the inner shaft 110b to the generator as shown FIG. 2E. In one embodiment, the temperature sensor 114 is arranged within the conduit 119a as shown in FIGS. 1H, 1l, 2A, 2D, and 2E in such a way the temperature sensor 114 can sense and read the temperature of the liquid metal 150 confined within the conduit 119a. Unlike the prior art device's temperature sensor which directly reads the temperature of the tissue, the temperature sensor 114 reads the liquid metal itself and returns the value to the generator for the temperature-controlled mode operation. In some embodiments without using the conduit space, the temperature sensor 114 is arranged at the distal end of the inner shaft 110b as shown in FIGS. 2B-2C.

Under the temperature controlled mode of the generator, for example, the operator initially set the temperature at 80 °C and monitors the temperature of the activated liquid metal from the temperature sensor 114 during the pre-defined time. When the temperature sensor 114 shows 60 °C or higher of the activated liquid metal device, then the operator can maintain the ablation until the pre-defined time.

For accurate reading the temperature of the activated liquid metal 150 itself during the ablation procedure, the temperature sensor 114 should be placed away from the tissue. As shown in FIGS. 2G and 2I, the temperature sensor 114 is configured to be attached as close as to the balloon or inside the covered portion or conduit 119a to avoid any direct tissue contact which may suggest the tissue temperature.

In some embodiments, the ablation catheter 100 can further comprise a temperature sensor mounted on the distal segment 119 of the shaft 110. The temperature sensor 114 can be arranged in the cavity of the cover element. The temperature sensor can be further formed as a thermocouple embedded in the distal segment of the flexible shaft, wherein a temperature sensing surface is formed as an endface of the distal end of the flexible shaft. In some embodiments, the temperature sensor can be formed as a thermocouple partially attached to the ring-shaped RF connector 115b to form a temperature sensing surface in the area of the ring-shaped RF connector 115b.

### Handle Portion

In one embodiment, the ablation catheter 100 further comprises the handle portion 130 as shown in FIGS. 1A and 1C for the operator. The handle portion 130 comprises a guidewire port 133, an electrical cable 132, and the injection port 131. The guidewire port 133 is connected to the guidewire lumen 111 for insertion of the guidewire 135. The electrical cable 132 is connected to the generator 160. The injection port 131 is connected to the fluid channel 112 for fluid communication with the liquid metal device 150. In one embodiment, a syringe is attached to the injection port 131 of the handle portion 130. The syringe contains a small or predefined volume of the liquid metal device 150.

In an alternative embodiment, the ablation catheter 100 can further comprise a handle portion arranged in the area of the distal end of the flexible shaft 110. The handle portion 130 can comprise a guidewire port, an electrical connection and an injection port. The guidewire port can be connected to a guidewire lumen adapted for insertion of a guidewire and formed in the flexible shaft. The electrical connection can be adapted to connect the RF connector 115 to an RF generator. The injection port can be connected to an injection lumen formed in the flexible shaft and adapted for instilling a liquid metal into a passageway. The injection port can be adapted for attachment of a syringe. Such an attachment can be formed for example by a Luer connector.

In some embodiments, the ablation catheter 100 can further comprise an injection port in the area of the proximal end of the shaft, that is in communication with the fluid channel of the flexible shaft. In some embodiments, the fluid channel is adapted to instill a liquid metal supplied to the fluid channel preferably via an injection port 131 into at least one passageway. The flexible shaft can further comprise an inflation lumen in communication with the inflatable balloon and adapted to supply or to discharge a fluid to/form the inflatable balloon.

### The Ablation Assembly

In one embodiment, the ablation catheter assembly configured to heat a passageway comprises a liquid metal contained in a syringe. The assembly further comprises the ablation catheter 100 comprising a flexible shaft having a fluid channel to instill the liquid metal into the passageway; and a RF connector 115 configured to connect RF energy to the liquid metal 150 thereby heating the passageway.

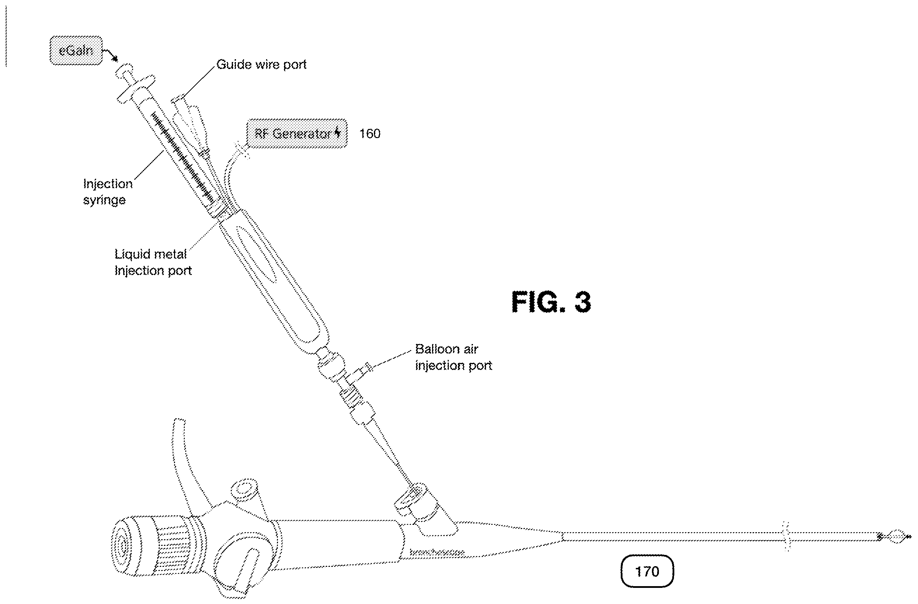

In some embodiments, the ablation catheter assembly comprises the ablation catheter 100 described herein. The assembly further comprises a flexible bronchoscope 170 comprising an instrument channel (sometimes also called a working channel). As shown in FIG. 3, the catheter device 100 is inserted through and travels through the instrument channel. The bronchoscope 170 may be relatively thin for traveling deep into the bronchial airways. In some embodiments, the bronchoscope 170 has a diameter of less than 4.0 mm; and in some cases, less than 2.0 mm. The bronchoscope 170 could also comprise other features. In some embodiments, the bronchoscope 170 further comprises a fluid channel for instilling or suctioning the liquid metal 150. The ablation assembly may further comprise an RF generator electrically coupled to the ablation catheter device 100. Finally, the ablation assembly may further comprise the liquid metal 150. In some embodiments, the liquid metal comprises gallium. In some embodiments, the liquid metal is E-Galn.

In some embodiments, the bronchoscope 170 can further comprise a fluid channel adapted to instill or suction fluid preferably into at least one passageway at a target site in a body organ. The ablation catheter assembly can further comprise an amount of liquid metal in electrical contact with the RF connector115.

According to a further aspect, an ablation kit comprises an RF ablation catheter according to the first aspect of the current invention or the ablation catheter assembly, and further comprises a container which comprises a liquid metal. The liquid metal can comprise gallium. The liquid metal can be provided to be liquid at 37° C.

### Liquid metal

To solve the problem of the requirement of exact delivery of the rigid metal electrode into the center of a target site in the peripheral lung legion, this invention uses a medical grade liquid metal device instead of using a solid electrode. The liquid metal device acts as an electrode through which RF ablation energy can be applied against the lung tumors. By injecting the liquid metal device into the target site, this will be conforming with the anatomical structures of the target site. Because of this conforming shape of the liquid metal device, there is less risk of damage to the surrounding regions. The liquid metal device is easily removed by aspiration suction without damaging the surrounding regions. Accordingly, the liquid metal 150 acts an independent and flexible electrode within the target site creating larger ablation area.

For more clearly defining herein, the liquid metal device could be stated as being a device suitable for treating cancer comprising one or more conductive metals in liquid form which conducts RF energy to target cancer masses. The preferred liquid metal is gallium based liquid metal. As a metal, it has high conductivity as high as metal, thermal conductivity high enough to be used for thermometer, excellent radio-opacity that can be used as a radio-contrast dye.

Furthermore, it has a very low melting point (15.5 °C), which keeps its liquid form at room temperature. Because of excellent radiopacity and high viscosity, the liquid metal injection into the target site of the bronchial tree is fully controllable under the fluoroscopic guidance. The injected liquid metal is gradually spreading from the proximal part to the distal without interruption according to the injected volume and pushing power. The operator is able to control the amount and extent of the liquid metal injection based on needs.

The pre-defined volume of the liquid metal 150 in the syringe may vary depending on the situation. In some embodiments, the liquid metal 150 has a volume of less than 1.0 ml; in some cases, less than 0.5 ml; and in some cases, less than 0.2 ml. In some embodiments, the syringe contains at least 0.05 ml of the liquid metal device 150. Most of the injected liquid metal is retrievable by bronchoscopic suction or natural expectoration over a few days. By means of fluoroscopic imaging analysis, about 82% of the injected liquid metal is able to be retrieved by active suction or passive expectoration. It is noteworthy that the liquidity of the liquid metal is the solution to eliminate the specific problems related with invasiveness such as puncture related problems, such as pneumothorax, arising from percutaneous approaches with rigid electrode needle, multi-tinned RF needles with poor controllability and unwanted damage coming from malposition electrode, etc.

The liquid metal 150 further comprises one or more conductive metals in liquid form.
Examples of liquid metals include gallium, and indium. In some embodiments, the liquid metal 150 comprises gallium. In some embodiments, liquid metal device 150 comprises indium. In some embodiments, the liquid metal 150 comprises a mixture of liquid metals, such as a combination of gallium, indium, and tin. One such example is "Galinstan", which is an alloy of gallium, indium, and tin. Another example is "eGaln", which is an alloy of gallium (75.5%) and indium (24.5%).

In some embodiments, the liquid metal can be heated to a range of 60° C to 80° C by applying an RF current. The liquid metal can comprise gallium. The liquid metal can be further provided to be liquid at 37° C. According to a further aspect of the invention, a liquid metal is delivered into a passageway of the body organ through the catheter. The liquid metal used in various embodiments of the invention is liquid at a body temperature, i.e., at 37°C. Preferably, it is also liquid at room temperature, i.e., at about 25 °C.

Metals are in general defined as materials that are capable of conducting electricity at a temperature of 0 Kelvin. The liquid metal used in the invention is pharmaceutically acceptable; i.e., non-toxic during the time of use of the metal and non-reactive. In one preferred embodiment, the liquid metal comprises gallium. Gallium has a melting point of 30 °C.

In a further preferred embodiment, the liquid metal is an alloy. Preferably, it is an eutectic alloy. In a further preferred embodiment, the liquid metal is an alloy comprising gallium, preferably at least 50 % by weight of gallium.

Gallium can readily alloy with most metals. So as an ingredient, it could be used to form many low-melting alloys with other metals, such as indium (In), bismuth (Bi), tin (Sn), lead (Pb), zinc (Zn), aluminum (Al) and so on. The melting points of the alloys are different depending on the constituents and the proportions. One embodiment is an alloy comprising 62-95% gallium, 5-22% indium, and 0-16% tin by weight.

EGaln (78.6% Ga and 21.4% In by weight) and Galinstan^{®} (68.5% Ga, 21.5% In, and 10.0% Sn by weight) are commonly and commercially available. They are eutectic mixtures. Taking EGaln as an example, it is fabricated by placing 78.6 wt% gallium and 21.4 wt% indium in a container, then heating and mixing them with a magnetic stirring apparatus or a glass pipette until thoroughly combined. Similar to gallium, bismuth could also comprise a series of low-melting alloys with Pb, Sn, Cd, Zn and In, etc.

### Target Ablation Size

The target ablation size may depend on the diameter and length of the target airway as well as the number of the subbranches of the target airway. For example, it shows that the less diameter of the target airway, the higher temperature of the ablation.

In some embodiments, the dashed line of FIG. 5B defines a target ablation area for RF ablation in which a portion of the target airway where two tumor masses are located. In some embodiments, the target ablation area may include a cluster of alveolar sacs arising from the terminal end of bronchial airway, but may exclude the lung pleura shown in FIG. 5B. In some embodiments, the target ablation area may be spherical or oval where, for example, the longest diameter of the ablation area may be about 7 cm and the shortest diameter of the ablation area may be about 4 cm, and the longest vertical diameter may be about 5-7 cm.

FIG. 5C shows a computer simulation result of each RF ablation in the same target airway. FIG. 5C(a) shows an x-ray fluoroscope image of the liquid metal device filled within the target airway. The fluoroscopic images showed the filling of the bronchial airway and its branches from various angles. In one experiment, the target airway was filled with a preferred liquid metal device which is eGaln and imaged by x-ray fluoroscopy. From these images, a 3D computer model of the eGaln filled bronchial airways was created. From tissue and RF energy modeling, this "tree" was simulated to create an ablation volume of 7 (long) x 4 by 4 cm egg-shaped volume of tissue ablation.

FIG. 5C(b) shows the ablation size when no conductive fluid filled within the target airway. FIG. 5C(c) shows the ablation size when conductive fluid of NaCl filled within the target airway. FIG. 5C(d) shows the ablation size when conductive fluid of gold nato-particle (AuNP)s filled within the target airway. Finally, the ablation size when the liquid metal device (E-Galn) filled within the target airway. FIG. 5D shows the target airway filled with liquid metal device has much larger ablation size.

### Procedure

The treatment method for ablating lung tumors use an approach through the patient's airway. The approach may be referred to as a transbronchial or endobronchial approach. The airway refers to the anatomical lumens through which air passes including the trachea, bronchi, and bronchioles. The system for this method may comprise (a) the ablation catheter, (b) the liquid metal device, (c) the bronchoscope or introducer sheath, and (d) the generator.

The treatment method may comprise inserting a bronchoscope into the target site. The ablation catheter 100 device is advanced to a target airway through the bronchoscope working channel. Then, the target airway is closed by inflating the occlusion balloon. Then, the liquid metal device is instilled into the target airway. RF electric current is applied to the RF connector 115. This RF current is transmitted through the liquid metal device to administer tissue-ablating RF energy to the tumor. The liquid metal device is suctioned out of the target site. Suctioning of the liquid material device could be done through the bronchoscope.

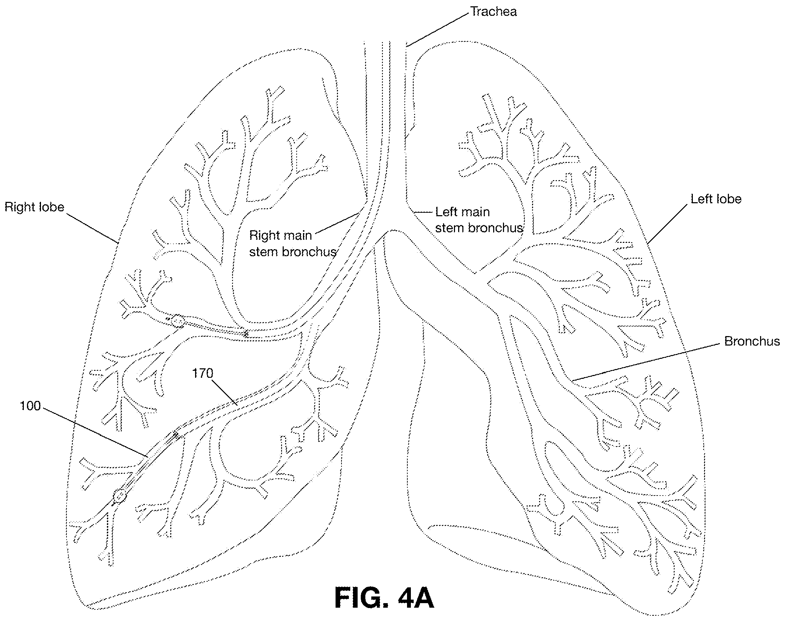

As shown in FIG. 4A, the ablation catheter 100 could be delivered to the target site as shown in FIGS. 4A-4B using a flexible bronchoscope 170 (4 mm in outer diameter and 2 mm working channel) as described herein. The ablation catheter device 100 is inserted through the instrument channel of the bronchoscope and the bronchoscope is advanced through the bronchial airways to the target site in the lung. At the target site, the ablation catheter device 100 is advanced out of the instrument channel of the bronchoscope and into the target airway.

When the ablation catheter is positioned at the proximal portion of the target airway, the occlusion balloon is inflated and locked to make the target airway a closed space to confine the later injected liquid metal device within the target airway as shown in FIGS. 6A-6B.

When the target airway is closed, the operator instills the liquid metal device into the closed target airway from the syringe as shown in FIG. 3. The syringe contains various predefined volumes of the liquid metal device such as 0.5 or 1.0 ml.

When the operator makes pressurized injection of the liquid metal device, the liquid metal device is gradually spreading from the proximal part to the distal of the target airway without interruption according to the injected volume and pushing power. The operator may be able to control the amount and extent of the liquid metal device based on needs under fluoroscopic guidance. For example, FIG. 4D shows that 0.75 ml of the liquid metal device is injected into the target airway by the operator under fluoroscopic guidance. The mean volume of the liquid metal device within a target airway may be about 0.5 ml. The mean volume, however, can be pre-determined depending on the anatomical variations and tumor mass locations.

In some embodiments, shown in FIG. 6E is the bronchoscope 170 with the ablation catheter 100 being delivered into a trunk bronchiole near the tumor masses. With the occluding balloon of the ablation catheter 100 inflated, the liquid metal device 150 is instilled into the bronchioles. The liquid metal device 150 is delivered out of the fluid channel 112 of the RF ablation catheter device 100. The liquid metal device 100 goes into the trunk bronchiole as well as three branches of the trunk bronchiole. When the target site is filled with the liquid metal device 150, the liquid metal device acts as a conformal electrode adjacent the tumor masses.

In this treatment method, the preferred liquid metal for injection is E-Galn. Since the liquid metal device (i.e., E-Galn) has adequate radiopacity, the device itself can be used as a radiocontrast agent. In addition, the liquid metal device (i.e., E-Galn) has high viscosity and a low melting point (15.5 °C) which keeps its liquid form at room temperature. Because of these attributes of E-Galn, the liquid metal device injection into the target site is fully controllable under fluoroscopic guidance. In some embodiments, the liquid metal comprises gallium.

By injecting the liquid metal device into the target airway, this will be conforming with the anatomical structures of the target airway as shown in FIG. 4D. Because of this conforming shape of the liquid metal device, it could serve as atraumatic conforming multiple RF electrodes. The injected liquid metal device acts an independent and flexible electrode within the target airway. In addition, the bronchial tree shape including the sub-branches as shown in FIG. 4D of the injected liquid metal device creates a much larger area of ablation than that of a single same bronchial tree without any side branches thereof. In some embodiments, the step of instilling comprises instilling the liquid metal into at least two branches of the bronchial airway. In some embodiments, the liquid metal is instilled only into bronchial airways having a diameter of less than 5 mm. In some embodiments, the liquid metal is instilled only into bronchial airways having a length of less than 10 cm.

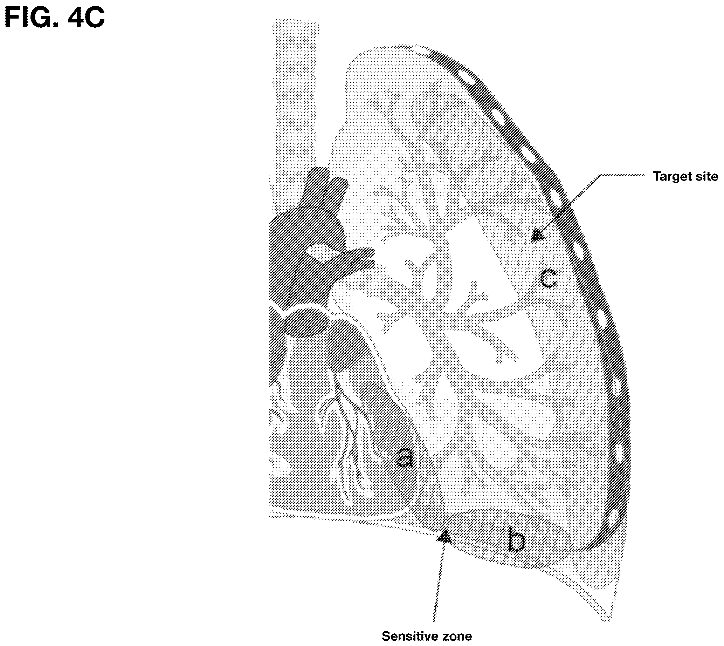

When the liquid metal device is injected into the target airway, a caution for distancing at least 5-10 mm of the distal tip of the liquid metal device from the pleura or other visceral organs in the sensitive zone may be advised to avoid unnecessary injury outside target site. Our computer simulation model also supports the distancing between the tip of the liquid metal device and the sensitive zone illustrated in FIG. 4C. Accordingly, the operator should keep it in mind that the liquid metal device injection into the small airway such as alveoli may be associated with not only the poor retrievability of the liquid metal device after the ablation but also the increased risk of unfavorable pleural or adjacent organ damage.

In some embodiments, the liquid metal device 150 is not instilled into any alveoli of the lung to avoid damage to the alveolar sac. The volume of the liquid metal device 150 may depend on various factors, such as the size of the tumor, location of the tumor, number of branches, etc. In some embodiments, the amount of liquid metal device 150 instilled is less than 2.0 ml; in some cases, less than 1.0 ml; and in some cases, less than 0.5 ml. In some embodiments, at least three bronchiole branches of the bronchial airways are instilled with the liquid metal device 150; and in some cases, at least five bronchiole branches.

When the liquid metal device is confined in a closed space within the target site, the operator selects the temperature-controlled mode of the RF generator with the desired temperature at 80 °C as ablation mode. Under this ablation mode, the RF connector 115 of the ablation device configured to allow RF current to pass through the injected liquid metal device in such a way the liquid metal device delivers radio frequency (RF) energy to ablate the tumors. The temperature sensor of the ablation device is configured only to read the activated liquid metal device, and create an RF ablation feedback loop.

Under the RF ablation feedback loop, the RF generator keeps delivering RF energy to the injected liquid metal through the RF connector until the injected liquid metal reaches to 60 °C which the temperature sensor directly reads from the injected liquid metal device. The effective ablation temperature may be defined as 40 °C, 50 °C, 60 °C, 70 °C, or 80 °C respectively depending on the anatomical structures.

Although a variety of ablation modes are applicable in each procedure, the temperature-controlled mode (set at 80 °C) was preferably used in the procedure by virtue of consistent and effective ablation. The ablation procedure may be terminated if there is any of the following conditions: (1) impedance rises over 250 Ω, (2) reaching out to the predetermined time (5, 10, 15 minutes according to the pre-defined procedure plan).

Thanks to its excellent bioavailability, Gallium-based liquid metal has been widely studied in the field of hyperthermic cancer treatment and artificial organs. For medial application, E-Galn can be used in the form of either "bulk material" or "microdroplet" through sonification process. Among them, microdroplet form is related with significant cytotoxicity reaction because it leads to high concentration of gallium and Indium ion release to the solution, in contrast to the bulk form of E-Galn.

In this procedure, the bulk-type E-Galn is used, and our experiments reaffirmed that serum gallium and indium concentrations can be almost negligible in pigs even with intentionally excessive amounts of E-Galn. This result is also consistent with other several studies investigating the direct inject of E-Galn into the tumor for the hyperthermic cancer treatment.

They also performed ISO guideline-directed E-Galn biocompatibility tests with the evidence that E-Galn is safe for intra-tissue injection. In our experiments, a single shot for the effective ablation usually needs E-Galn of less than 1 ml. And most of them (about 70-90%) are able to be directly retrieved by bronchoscopic suction or natural expectoration thereafter. This residual bronchial electrode (E-Galn) amount corresponds to the about hundreds of times less than the amount of E-Galn for intra-tumoral injections in those studies assuming the same body weight. In our experiments, the residual liquid metal device in the target site is not associated with any significant problems in lung either. Regarding indium toxicity, it is well-known that indium is toxic to the lungs, but this occurs only when indium is distributed in the lungs in the form of inhaled gas. This is not the case of the ablation in this procedure.

FIG. 4E shows the target site where most of the injected liquid metal is retrieved by bronchoscope suction immediately after the ablation procedure or natural expectoration over a few days unless the injected liquid metal device is entrapped by the small airway such as alveolus. By means of fluoroscopic imaging analysis, about 82% of the injected liquid metal may be able to be retrieved by active suction or passive expectoration.

The descriptions and examples given herein are intended merely to illustrate the invention and are not intended to be limiting. Each of the disclosed aspects and embodiments of the invention may be considered individually or in combination with other aspects, embodiments, and variations of the invention. In addition, unless otherwise specified, the steps of the methods of the disclosure are not confined to any particular order of performance.

Any use of the word "or" herein is intended to be inclusive and is equivalent to the expression "and/or," unless the context clearly dictates otherwise. As such, for example, the expression "A or B" means A, or B, or both A and B. Similarly, for example, the expression "A, B, or C" means A, or B, or C, or any combination thereof.

## Claims

1. A radiofrequency ,RF, ablation catheter (100), configured to heat a passageway filled with a liquid metal, the ablation catheter comprising:
a flexible shaft (110) extending between a distal end and a proximal end, said flexible shaft having a fluid channel (112) configured to instill said liquid metal into the passageway
an inflatable balloon (120) mounted on said flexible shaft; and
an RF connector (115);
wherein a portion of said flexible shaft that is distal to said inflatable balloon is defined as a distal segment of said flexible shaft; and
wherein said RF connector is located at said distal segment and is configured for connecting RF energy to said liquid metal thereby heating said passageway, **characterised in that** said liquid metal is comprised within said fluid channel.

2. The RF ablation catheter according to claim 1, wherein said distal segment forms a conduit (119a) inside said distal segment in such a way said conduit conveys said liquid metal from said fluid channel.

3. The RF ablation catheter according to claim 2, wherein said RF connector is located within said conduit in a recessed manner.

4. The RF ablation catheter according to claim 1, wherein said RF connector is arranged at said proximal end of said distal segment directly neighbouring said inflatable balloon or wherein said RF connector is an electrically conductive (115a) connected to a RF generator; or wherein said ablation catheter further comprises a temperature sensor (114) mounted on said distal segment of said flexible shaft and configured to read the temperature of said liquid metal.

5. The RF ablation catheter according to claim 2, wherein said temperature sensor is arranged within said conduit in a recessed manner; or wherein said conduit is made of non-conductive material.

6. The RF ablation catheter according to claim 1, wherein said passageway is a bronchial airway of the lung

7. An ablation catheter assembly, configured to heat a passageway, the assembly comprising:
said liquid metal contained in a syringe, said syringe, and
the RF ablation catheter according to claim 1.

8. The ablation catheter assembly of claim 7 further comprising:
a flexible bronchoscope (170) comprising a working channel configured for said ablation catheter to travel through said fluid channel.

9. The ablation catheter assembly according to claim 7, said ablation catheter further comprises an RF generator electrically coupled to said RF connector

10. The ablation catheter assembly according to claim 7, wherein said passageway is a bronchial airway of the lung, or wherein said liquid metal comprises a gallium.

11. The ablation catheter assembly according to claim 7, wherein said liquid metal is E-Galn.

12. The ablation catheter assembly according to claim 7, wherein said distal segment forms a conduit inside said distal segment in such a way , that said conduit is configured to convey said liquid metal from said fluid channel.

13. The ablation catheter assembly according to claim 7, wherein said RF connector is located within said conduit in a recessed manner.

14. The ablation catheter assembly according to claim 7, wherein said conduit is made of non-conductive material.

## Patentansprüche

1. Radiofrequenz-(HF-)Ablationskatheter (100), der zum Erwärmen eines mit einem flüssigen Metall gefüllten Durchgangs konfiguriert ist, wobei der Ablationskatheter Folgendes umfasst:
einen flexiblen Schaft (110), der sich zwischen einem distalen Ende und einem proximalen Ende erstreckt, wobei der flexible Schaft einen Fluidkanal (112) aufweist, der zum Eintröpfeln des flüssigen Metalls in den Durchgang konfiguriert ist;
einen aufblasbaren Ballon (120), der an dem flexiblen Schaft montiert ist; und
einen HF-Verbinder (115);
wobei ein Abschnitt des flexiblen Schafts, der sich distal zu dem aufblasbaren Ballon befindet, als distales Segment des flexiblen Schafts definiert ist; und
wobei sich der HF-Verbinder an dem distalen Segment befindet und zum Verbinden von HF-Energie mit dem flüssigen Metall konfiguriert ist, wodurch der Durchgang erwärmt wird, **dadurch gekennzeichnet, dass** das flüssige Metall innerhalb des Fluidkanals umfasst ist.

2. HF-Ablationskatheter nach Anspruch 1, wobei das distale Segment eine Leitung (119a) innerhalb des distalen Segments derart ausbildet, dass die Leitung das flüssige Metall aus dem Fluidkanal fördert.

3. HF-Ablationskatheter nach Anspruch 2, wobei sich der HF-Verbinder versenkt innerhalb der Leitung befindet.

4. HF-Ablationskatheter nach Anspruch 1, wobei der HF-Verbinder an dem proximalen Ende des distalen Segments direkt benachbart zu dem aufblasbaren Ballon angeordnet ist oder wobei der HF-Verbinder ein mit einem HF-Generator verbundener elektrischer Leiter (115a) ist; oder wobei der Ablationskatheter ferner einen Temperatursensor (114) umfasst, der an dem distalen Segment des flexiblen Schafts montiert und zum Ablesen der Temperatur des flüssigen Metalls konfiguriert ist.

5. HF-Ablationskatheter nach Anspruch 2, wobei der Temperatursensor versenkt innerhalb der Leitung angeordnet ist; oder wobei die Leitung aus nichtleitendem Material hergestellt ist.

6. HF-Ablationskatheter nach Anspruch 1, wobei der Durchgang ein bronchialer Luftweg der Lunge ist.

7. Ablationskatheterbaugruppe, die zum Erwärmen eines Durchgangs konfiguriert ist, wobei die Baugruppe Folgendes umfasst:
das in einer Spritze enthaltene flüssige Metall, die Spritze und den HF-Ablationskatheter nach Anspruch 1.

8. Ablationskatheterbaugruppe nach Anspruch 7, ferner umfassend:
ein flexibles Bronchoskop (170), umfassend einen Arbeitskanal, der zum Bewegen des Ablationskatheters durch den Fluidkanal konfiguriert ist.

9. Ablationskatheterbaugruppe nach Anspruch 7, wobei der Ablationskatheter ferner einen HF-Generator umfasst, der elektrisch an den HF-Verbinder gekoppelt ist.

10. Ablationskatheterbaugruppe nach Anspruch 7, wobei der Durchgang ein bronchialer Luftweg der Lunge ist; oder wobei das flüssige Metall ein Gallium umfasst.

11. Ablationskatheterbaugruppe nach Anspruch 7, wobei das flüssige Metall E-Galn ist.

12. Ablationskatheterbaugruppe nach Anspruch 7, wobei das distale Segment eine Leitung innerhalb des distalen Segments derart ausbildet, dass die Leitung zum Fördern des flüssigen Metalls aus dem Fluidkanal konfiguriert ist.

13. Ablationskatheterbaugruppe nach Anspruch 7, wobei sich der HF-Verbinder versenkt innerhalb der Leitung befindet.

14. Ablationskatheterbaugruppe nach Anspruch 7, wobei die Leitung aus nichtleitendem Material hergestellt ist.

## Revendications

1. Cathéter d'ablation par radiofréquence, RF (100), configuré pour chauffer un passage rempli d'un métal liquide, le cathéter d'ablation comprenant :
une tige flexible (110) se prolongeant entre une extrémité distale et une extrémité proximale, ladite tige flexible présentant un canal de fluide (112) configuré pour instiller ledit métal liquide dans le passage ;
un ballonnet gonflable (120) monté sur ladite tige flexible ; et
un connecteur RF (115) ;
dans lequel une partie de ladite tige flexible qui est distale par rapport audit ballon gonflable est définie comme un segment distal de ladite tige flexible ; et
dans lequel ledit connecteur RF est situé au niveau dudit segment distal et est configuré pour connecter l'énergie RF audit métal liquide, chauffant ainsi ledit passage, **caractérisé en ce que** ledit métal liquide est compris dans ledit canal de fluide.

2. Cathéter d'ablation RF selon la revendication 1, dans lequel ledit segment distal forme un conduit (119a) à l'intérieur dudit segment distal de telle manière que ledit conduit transporte ledit métal liquide depuis ledit canal de fluide.

3. Cathéter d'ablation RF selon la revendication 2, dans lequel ledit connecteur RF est situé à l'intérieur dudit conduit de manière encastrée.

4. Cathéter d'ablation RF selon la revendication 1, dans lequel ledit connecteur RF est disposé au niveau de ladite extrémité proximale dudit segment distal, directement à proximité dudit ballonnet gonflable, ou dans lequel ledit connecteur RF est un connecteur électriquement conducteur (115a) connecté à un générateur RF ; ou dans lequel ledit cathéter d'ablation comprend également un capteur de température (114) monté sur ledit segment distal de ladite tige flexible et configuré pour lire la température dudit métal liquide.

5. Cathéter d'ablation RF selon la revendication 2, dans lequel ledit capteur de température est disposé à l'intérieur dudit conduit de manière encastrée ; ou dans lequel ledit conduit est constitué d'un matériau non conducteur.

6. Cathéter d'ablation RF selon la revendication 1, dans lequel ledit passage est une voie aérienne bronchique du poumon.

7. Ensemble de cathéter d'ablation, configuré pour chauffer un passage, l'ensemble comprenant :
ledit métal liquide contenu dans une seringue, ladite seringue et le cathéter d'ablation RF selon la revendication 1.

8. Ensemble de cathéter d'ablation selon la revendication 7, comprenant également :
un bronchoscope flexible (170) comprenant un canal de travail configuré pour que ledit cathéter d'ablation se déplace à travers ledit canal de fluide.

9. Ensemble de cathéter d'ablation selon la revendication 7, ledit cathéter d'ablation comprenant également un générateur RF couplé électriquement audit connecteur RF.

10. Ensemble de cathéter d'ablation selon la revendication 7, dans lequel ledit passage est une voie aérienne bronchique du poumon ; ou dans lequel ledit métal liquide comprend du gallium.

11. Ensemble de cathéter d'ablation selon la revendication 7, dans lequel ledit métal liquide est de l'E-Galn.

12. Ensemble de cathéter d'ablation selon la revendication 7, dans lequel ledit segment distal forme un conduit à l'intérieur dudit segment distal de telle manière que ledit conduit est configuré pour transporter ledit métal liquide depuis ledit canal de fluide.

13. Ensemble de cathéter d'ablation selon la revendication 7, dans lequel ledit connecteur RF est situé à l'intérieur dudit conduit de manière encastrée.

14. Ensemble de cathéter d'ablation selon la revendication 7, dans lequel ledit conduit est constitué d'un matériau non conducteur.
